(19) Europäisches Patentamt

European Patent Office

Office européen des brevets

(11) **EP 1 514 512 A1**

(12) **EUROPEAN PATENT APPLICATION**

(43) Date of publication:
**16.03.2005 Bulletin 2005/11**

(51) Int Cl.⁷: **A61B 5/03**

(21) Application number: **04255053.3**

(22) Date of filing: **20.08.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL HR LT LV MK** | (72) Inventor: **Rosenberg, Meir**<br>**Newton MA 02459 (US)** |
| (30) Priority: **22.08.2003 US 646108** | (74) Representative: **Mercer, Christopher Paul et al**<br>**Carpmaels & Ransford,**<br>**43-45 Bloomsbury Square**<br>**London WC1A 2RA (GB)** |
| (71) Applicant: **Codman & Shurtleff, Inc.**<br>**Raynham, Massachusetts 02767-0350 (US)** | |

(54) **Intra-ventricular pressure sensing catheter**

(57)     An intra-ventricular pressure sensor device is provided that includes a catheter having a first lumen for receiving fluid flow therethrough, and a second separate, fluid-filled, fluid-impermeable lumen extending between a pressure-sensitive component that is adapted to be exposed to an external pressure source, and a pressure sensor that is effective to measure pressure of the external pressure source in response to displacement of the pressure-sensitive component. The intra-ventricular pressure sensor device is particularly advantageous in that it allows a direct measurement of a patient's ventricular pressure to be obtained.

FIG. 2

**Description**

FIELD OF THE INVENTION

The present invention relates to a catheter device having a pressure sensor disposed therein.

BACKGROUND OF THE INVENTION

Hydrocephalus is a neurological condition that is caused by the abnormal accumulation of cerebrospinal fluid (CSF) within the ventricles, or cavities, of the brain. CSF is a clear, colorless fluid that is primarily produced by the choroid plexus and surrounds the brain and spinal cord. CSF constantly circulates through the ventricular system of the brain and is ultimately absorbed into the bloodstream. CSF aids in the protection of the brain and spinal cord. Because CSF keeps the brain and spinal cord buoyant, it acts as a protective cushion or "shock absorber" to prevent injuries to the central nervous system.

Hydrocephalus, which affects children and adults, arises when the normal drainage of CSF in the brain is blocked in some way. Such blockage can be caused by a number of factors, including, for example, genetic predisposition, intraventricular or intracranial hemorrhage, infections such as meningitis, head trauma, or the like. Blockage of the flow of CSF consequently creates an imbalance between the amount of CSF produced by the choroid plexus and the rate at which CSF is absorbed into the bloodstream, thereby increasing pressure on the brain, which causes the ventricles to enlarge.

Hydrocephalus is most often treated by surgically inserting a shunt system that diverts the flow of CSF from the ventricle to another area of the body where the CSF can be absorbed as part of the circulatory system. Shunt systems come in a variety of models, and typically share similar functional components. These components include a ventricular catheter which is introduced through a burr hole in the skull and implanted in the patient's ventricle, a drainage catheter that carries the CSF to its ultimate drainage site, and optionally a flow-control mechanism, e.g., shunt valve, that regulates the one-way flow of CSF from the ventricle to the drainage site to maintain normal pressure within the ventricles. The ventricular catheter typically contains multiple holes or pores positioned along the length of the ventricular catheter to allow the CSF to enter into the shunt system. To facilitate catheter insertion, a removable rigid stylet, situated within the lumen of the ventricular catheter, is used to direct the catheter toward the desired targeted location. Alternatively, or in addition, blunt tip brain cannulas and peel-away sheaths have been used to aid placement of the catheters.

One common problem encountered with the use of ventricular catheters is the difficulty in measuring the pressure within the patient's ventricle. Measurement of intra-ventricular pressure is currently achieved using two techniques. One technique involves placing a telemetrically communicating miniaturized pressure sensor in the ventricles. Such pressure sensors however, require a high degree of miniaturization and are therefore sensitive to environmental degradation. The other technique involves placing a pressure sensor that communicates with the cerebrospinal fluid in line and distal to the ventricles. As the pressure drop across the catheter is negligible, the sensor can measure pressure that resembles the intra-ventricular pressure. While this technique is advantageous in that it allows the use of a relatively large sensor, catheter blockage can impede the pressure sensed by the sensor, thus preventing an accurate measurement of intra-ventricular pressure from being obtained.

Accordingly, there remains a need for a catheter having a pressure sensor that is effective to accurately measure a patient's ventricular pressure.

SUMMARY OF THE INVENTION

The present invention generally provides a pressure sensor device having an elongate catheter with a first lumen that is adapted to accommodate fluid flow therethrough, and a second, separate, fluid-filled, fluid-impermeable, sealed lumen. The second lumen extends between a pressure-sensitive component that is adapted to be exposed to an external pressure source, and a pressure sensor that is effective to measure pressure of the external pressure source in response to displacement of the pressure-sensitive component. The first lumen can include at least one fluid-entry port formed through the sidewall of the catheter at a location that is distal to the proximally-located pressure sensor for receiving fluid flow therethrough.

In one embodiment, the pressure-sensitive component is disposed at a distal end of the second lumen, and the pressure sensor is coupled to a proximal end of the second lumen. The pressure-sensitive component can include a first surface in contact with fluid within the second lumen, and a second, opposed surface adapted to be exposed to an external pressure source. In an exemplary embodiment, the pressure-sensitive component is a flexible membrane which is preferably disposed across an opening formed in the sidewall of the catheter. The flexible membrane can have a compliance that is in the range of about 0.05 $\mu$L/mmHg to 2 $\mu$L/mmHg.

In yet another embodiment of the present invention, an intra-ventricular catheter is provided having an elongate member with a first lumen that is adapted to accommodate fluid flow therethrough, and a second, fluid-sealed lumen having a pressure sensor coupled to a flexible membrane disposed at a distal end of the catheter and that is adapted to respond to intra-ventricular pressure changes when the catheter is implanted within a patient's ventricle such that direct pressure readings of the intra-ventricular pressure can be measured. The pressure sensor can be coupled to a proximal end of the second, fluid-sealed lumen. In an alternative embodiment, the flexible membrane can be disposed at a mid-portion of the catheter such that it is adapted to respond to intra-parenchymal pressure changes.

The present invention also provides a method for measuring intra-ventricular pressure using a ventricular catheter having a first lumen that is adapted to accommodate fluid flow therethrough, and a second, fluid-sealed, fluid-impermeable lumen extending between a distal, pressure-sensitive member adapted to respond to pressure changes in a patient's ventricle, and a proximal pressure sensor adapted to measure the pressure changes. The method includes the steps of implanting the ventricular catheter in a patient's ventricle such that the pressure-sensitive member is disposed within the ventricle and the pressure sensor is disposed at a location outside of the ventricle, and obtaining at least one reading of the pressure within the patient's ventricle.

In other aspects of the present invention, a method for manufacturing an intra-ventricular pressure sensor device is provided. The method includes the step of forming a catheter having a first lumen adapted to receive fluid flow therethrough, and a second lumen extending between a proximal, pressure sensor and a distal end in communication with an opening formed in a sidewall of the catheter. The second lumen is then filled with fluid, and a solvent-based solution is sprayed over the opening formed in the sidewall of the catheter to form a flexible membrane that is effective to seal the fluid within the second lumen in the catheter. The solvent-based solution should be effective to adhere to the catheter. Preferably, all voids in the second lumen are removed from the second lumen after the second lumen is filled with fluid, and prior to spraying the solution onto the catheter.

BRIEF DESCRIPTION OF THE DRAWINGS

The invention will be more fully understood from the following detailed description taken in conjunction with the accompanying drawings, in which:

FIG. 1 is a side view of one embodiment of an intra-ventricular pressure sensor system according to the present invention;

FIG. 2 is a cross-sectional view of the intra-ventricular pressure sensor system shown in FIG. 1; and

FIG. 3 is a side view of another embodiment of an intra-ventricular pressure sensor system according to the present invention.

DETAILED DESCRIPTION OF THE INVENTION

The present invention generally provides an intra-ventricular pressure sensor device that includes a catheter having a first lumen for receiving fluid flow therethrough, and a second, separate, fluid-filled, fluid-impermeable, sealed lumen extending between a pressure-sensitive component that is adapted to be exposed to an external pressure source, and a pressure sensor that is effective to measure pressure of the external pressure source in response to displacement of the pressure-sensitive component. An incompressible fluid is disposed within the second lumen. This allows the pressure sensed by the pressure-sensitive member to be directly translated to the pressure sensor, thereby allowing the measurement of pressure by a sensing element which is not in direct communication with the pressure of interest. The intra-ventricular pressure sensor device is particularly advantageous in that it allows a direct measurement of a patient's ventricular pressure to be obtained.

While the device is described as a ventricular catheter that is used to measure the pressure in a patient's ventricles, a person skilled in the art will appreciate that the device can be used for a variety of medical procedures to measure the pressure in a variety of cavities. By way of non-limiting example, the device can be modified to measure intra-parenchymal pressure.

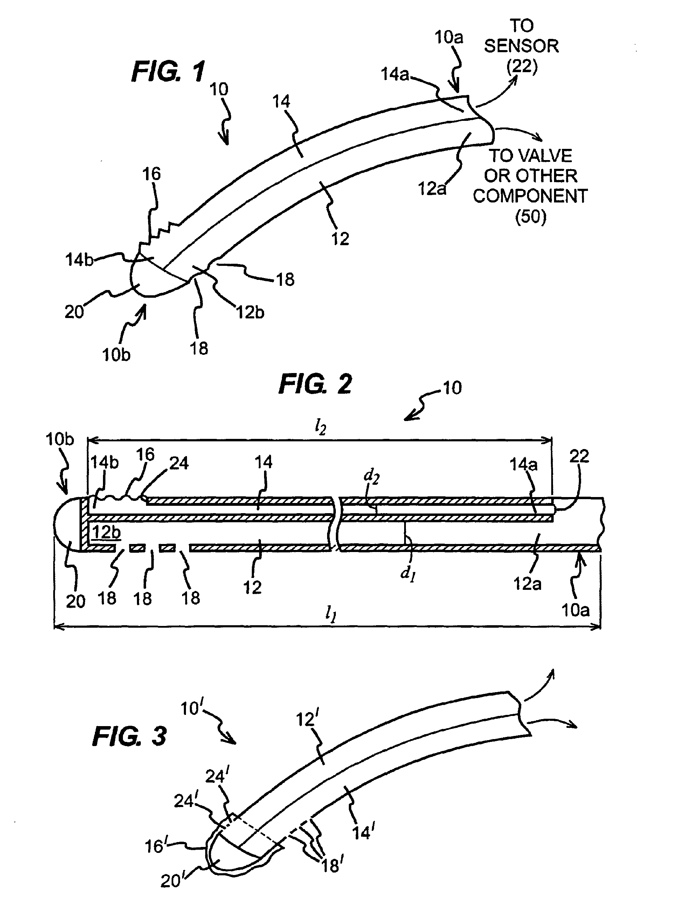

FIGS. 1 and 2 illustrate an exemplary embodiment of an intra-ventricular pressure sensor device having an elongate catheter 10 with a proximal end 10a, a distal end 10b, and at least one inner lumen, e.g., lumen 12, extending therethrough that is adapted to accommodate fluid flow therein. The catheter 12 also includes a second lumen 14 that is in fluid isolation from the first lumen 12, and that is fluid impermeable. The second lumen 14 is a fluid-filled, sealed lumen that includes a proximal end 14a that is in fluid communication with a sensor 22, and a distal end 14b having a pressure-sensitive component, e.g., flexible membrane 16, that is adapted to be displaced by an external pressure source. In use, the pressure sensor 22 is effective to measure the pressure of the external pressure source in response to displacement of the pressure-sensitive component 16.

The elongate catheter 10 can have a variety of configurations, but it is preferably a semi-flexible or flexible elongate member having proximal and distal ends 10a, 10b with at least one inner lumen 12, 14 extending therebetween. The first inner lumen 12 is adapted to accommodate fluid flow therethrough, and thus it can include an open proximal end 12a that can be connected to another medical device, such as a valve for controlling fluid flow from the catheter. The distal end 10b of the catheter 10, on the other hand, can either be open or closed, but preferably it is closed and includes a blunt end cap 20 formed thereon to facilitate insertion and/or imaging of the device 10. The end cap 20 is advantageous in that it facilitates insertion of the device 10 and it prevents the distal tip of an insertion device, such as a rigid stylet (not shown), from penetrating the distal end 10b of the catheter 10. The end cap 20 can also optionally be formed from a radio-opaque material to facilitate imaging of the catheter 10. The catheter 10 can also include one or more fluid-entry ports 18 formed in the sidewall thereof and in communication with the first lumen 12 to allow fluid to flow into the catheter 10 and through the first lumen 10.

The dimensions of the catheter 10 can vary depending on the intended use, but preferably the catheter 10 has a length $l$ that is sufficient to allow at least the distal portion 10b of the catheter 10 to be implanted in a patient's ventricles, while the proximal portion 10a can extend therefrom to connect, for example, to another medical device such as a valve. The length $l$ of the catheter 10 should also be configured to optimize the ability of the pressure sensor 22 to obtain an accurate reading of the intra-ventricular pressure, as will be discussed in more detail below. In an exemplary embodiment, the length l is in the range of about 5 cm to 20 cm, and more preferably it is about 15 cm.

A person skilled in the art will appreciate that the catheter 10 can have virtually any configuration, shape, and size, and that it can be adapted for use in a variety of medical procedures. Moreover, the catheter 10 can be formed from a variety of materials. In an exemplary embodiment, however, the catheter 10 is formed from a flexible, biocompatible material. Suitable materials include, for example, polymers such as silicones, polyethylene, and polyurethanes, all of which are known in the art. The catheter 10 can also optionally be formed from a radio-opaque material.

As previously stated, the catheter 10 also includes a second lumen 14 that is in fluid isolation from the first lumen 12. The second lumen 14 extends from the proximal end 10a of the catheter 10 to a position distal to the proximal end 10a. The proximal end 14a of the second lumen 14 is coupled to a pressure sensor 22, and the distal end 14b of the second lumen 14 is in communication with a pressure-sensitive component, such as a flexible membrane 16, that is disposed over at least one discontinuity or opening 24 (FIG. 2) formed in a sidewall of the catheter 10 adjacent to the distal end 10b of the catheter 10. The pressure-sensitive component will be discussed in more detail below.

The position at which the second lumen 14 terminates with respect to the distal end 10b of the catheter can vary depending on the intended use. For example, where the catheter 10 is configured to measure the intra-ventricular pressure, the second lumen 14 preferably terminates at or adjacent the distal end 10b of the catheter. Alternatively, where the catheter 10 is configured to measure the intra-parenchymal pressure, the second lumen 14 can terminate at a mid-point on the catheter 10 such that the pressure-sensitive member 16 will be in contact with the parenchyma tissue when the device 10 is implanted. Thus, while the catheter 10 is shown and described for use in measuring intra-ventricular pressure, a person skilled in the art will appreciate that the device can easily be modified to measure the intra-parenchymal pressure, as well as the pressure within a variety of other cavities in a patient's body.

The dimensions of the second lumen 14 and the opening(s) 24 can vary, but they should be adapted to optimize the system's performance. In particular, the length $l_2$ and diameter $d_2$ of the second lumen 14 will affect the frequency response of the system, which indicates the time it takes for the system to obtain a pressure reading. For example, a lumen having a relatively long length will delay the amount of time it takes to obtain a reading. Thus, the length $l_2$ and diameter $d_2$ of the second lumen 14 should be adapted to provide a high frequency response, and in an exemplary embodiment the length $l_2$ and diameter $d_2$ of the second lumen 14 should be configured to produce a frequency response that is greater than about 20 Hz, and more preferably that is at least about 100 Hz. While this will be discussed in more detail below, in general the diameter $d_2$ of the second lumen 14 is preferably smaller than a diameter $d_1$ of the first lumen 12, and more preferably the diameter $d_2$ of the second lumen 14 is in the range of about 0.1 mm to 0.3 mm, and the diameter $d_1$ of the first lumen 12 is in the range of about 1 mm to 2 mm. Since the second lumen 14 is sealed, the lumen 14 is adapted to retain a predetermined volume of fluid. While this volume can vary for the same aforementioned reasons, in an exemplary embodiment the volume is in the range of about 1 μL to 10 μL.

The relatively small diameter $d_2$ and length $l_2$ of the second lumen 14 will also minimize the affect of the flexibility of the catheter 10 on the volume of the fluid contained in the second lumen 14. Bending of the catheter 10, as well as expansion or compression due to thermal or pressure variations, can undesirably affect the volume of fluid within the second lumen 14. The use of a second lumen 14 that has a relatively small diameter $d_2$ and length $l_2$, in combination with a compliant pressure-sensitive member 16, will minimize the effect of physical changes that may occur to the catheter 10 during use of the device.

The fluid that is disposed within the second lumen 14 can vary, but preferably the fluid is an incompressible, biocompatible fluid that cannot diffuse or penetrate into the walls of the catheter 10. Since the fluid is used to transfer a pressure received by the pressure-sensitive member 16 to the pressure sensor 22, a low viscosity fluid is preferred. Moreover, the fluid is also preferably a thermally stable fluid with extremely low thermal expansion coefficient. This will minimize the effect of thermal changes in the body on the volume of fluid in the second lumen 14. In an exemplary embodiment, the fluid in the second lumen 14 has a viscosity that is in the range of about 5 cs to 20 cs, and more preferably the fluid is a biocompatible silicone fluid. Suitable fluids include, for example, polydimethylsiloxane, which can be obtained from Dow Coming Corporation, of Midland, Michigan.

As stated above, the opening 24 that extends into the second lumen 14 is coupled to a pressure-sensitive member, which can have a variety of configurations. The pressure-sensitive member should, however, be adapted to respond to pressure changes in an environment surrounding the catheter 10, e.g., changes in the intra-ventricular pressure when the catheter 10 is implanted within a patient's ventricles. In the embodiment shown in FIGS. 1 and 2, the pressure-sensitive member is a flexible membrane 16 that is disposed over and extends across the opening(s) 24 in the second lumen 14. The membrane 16 is effective to seal the fluid within the second lumen 14, and to transfer a pressure signal, via the fluid, through the lumen 14 to the pressure sensor 22. Thus, in order to provide an accurate pressure reading, the membrane 16 should have a compliance that allows it to respond to external pressure changes. For example, an increase in a patient's intra-ventricular pressure will apply a force to the membrane 16, and the membrane 16 should be able to transfer that force to the fluid disposed within the second lumen 14. Since the lumen 14 is sealed, in order to maintain an equilibrium in the system, the force is transferred to the pressure sensor 22 at the proximal end 14a of the lumen 14, thus allowing the patient's intra-ventricular pressure to be obtained. The compliance of the membrane 16 will therefore affect the ability of the sensor 22 to obtain an accurate pressure reading. Where a small, relatively stiff membrane is utilized, a large force is required to displace the membrane and thereby transfer the pressure to the pressure sensor. In other words, a membrane which is extremely stiff will isolate the internal fluid from the external pressure and changes in the external environment will not be sensed in the internal fluid. Thus, it is desirable to provide a flexible membrane, e.g., a compliant membrane, that requires a small amount of force to create a shift in the equilibrium, and thus transfer a pressure signal to the pressure sensor. The compliance of the membrane should therefore be adjusted to provide accurate pressure readings with the difference between the actual pressure and the measured pressure preferably being less than 1 cm $H_2O$. The compliance of the membrane 16 can be altered by adjusting parameters such as the material and/or the shape and size of the membrane 16. In an exemplary embodiment, the membrane 16 has a compliance that is greater than a compliance of the sensor, and more preferably the membrane has a compliance that is in the range of about 0.05 µL/mmHg to 2 µL/mmHg.

The material used to form the membrane 16 can vary, and a variety of techniques can be used to attach the membrane 16 to the catheter 10. By way of non-limiting example, suitable materials include polyurethane, silicone, solvent-based polymer solutions, and any other polymer that will adhere to the catheter. In an exemplary embodiment, the membrane 16 is spray-coated onto the catheter 10 after the lumen 14 is filled with fluid and all voids or air bubbles have been removed.

In an alternative embodiment, shown in FIG. 3, the membrane can be in the form of a sleeve 16' that is disposed around a distal portion 10b' of the catheter 10'. The sleeve 16', which is formed around the distal end 10b' of the catheter 10', is in fluid communication with the second inner lumen 14' via one or more openings 24' formed in the sidewall of the catheter 10'. The sleeve 16' should, however, be isolated from the fluid-entry ports 18' that extend into the first lumen, and thus the fluid-entry ports 18' in this embodiment are preferably positioned more proximal from the distal end 10b' of the catheter 10'. The sleeve 16' configuration is particularly advantageous in that it enables the use of a relatively large membrane, thus increasing the compliance of the membrane. A highly compliant membrane will decrease the pressure drop across the membrane, thereby improving the precision of the pressure sensing device.

Referring back to FIGS. 1 and 2, the proximal end 14a of the second lumen 14 is coupled to a pressure sensor 22 which is effective to measure the pressure transferred from the flexible membrane 16 via the fluid disposed within the second lumen 14. The pressure sensor 22 can be coupled to any portion of the proximal end 14a of the second lumen 14. For example, in the embodiment shown in FIG. 2, the sensor 22 is disposed across the open proximal end 14a of the second lumen 14 to seal the fluid within the lumen 14. The sensor 22 is flexible to respond to pressure transferred through the fluid from the membrane 16. A variety of sensors are available and can be used with the present invention including, for example, piezoelectric materials and capacitive sensors. By way of non-limiting example, suitable sensors can be obtained from Millar, of Houston, Texas.

While a variety of pressure sensors 22 can be used, the sensor 22 is preferably formed from a relatively stiff material, and it has a low compliance, thus increasing the frequency response of the system. In an exemplary embodiment, the sensor 22 has a compliance that is in the range of about 0.1 µL/mmHg to 0.02 µL/mmHg. In use, the pressure sensor 22 is coupled to electronics that are known in the art and that are effective to translate the changes in the material into a pressure measurement.

A person skilled in the art will appreciate that the properties of each component of the catheter 10 are co-dependent, and that the relationship between the components will need to be considered in determining the appropriate configuration for each individual component. In an exemplary embodiment, the components should be configured to enable the sensor 22 to obtain an accurate reading of a patient's intra-ventricular pressure. It is understood, however, that the measured pressure reading will vary slightly from the actual intra-ventricular pressure due to the system compliance and resistance, which relies on a number of factors including the fluid compressibility, the tubing compliance, and the sensor flexibility. The compliance and resistance of the system should therefore be minimized, as any compliance and/or resistance within the system will distort the acquired signal. This can be achieved by adapting the system such that the frequency response of the system is greater than 100 Hz (i.e., the time constant is less than 0.01 seconds), which exceeds the frequency content of the physiological signal of interest. Based on this factor, the dimensions of the system can be selected such that:

$$\frac{512\mu\ell^2 a^2}{E d^2 (a^2 - d^2)} < 0.01$$

where $\mu$ is the fluid viscosity, $l$ is the length of the catheter 10, $a$ is the inner diameter of the second lumen plus the twice the thickness of the wall that separates the first and second lumens (i.e., in essence, the outer diameter of the second lumen), E is the modulus of elasticity of the catheter, and d is the inner diameter of the second lumen. An exemplary embodiment of a catheter that meets these requirements is illustrated in Example 1.

Example 1

A silicone catheter having a first lumen and a second lumen that is filled with silicone fluid has the following properties:

$$\mu = 10^{-3}$$

$$\ell = 0.1 m$$

$$a = 1.10^{-3} m$$

$$d = 0.5.10^{-3} m$$

$$E = 5000 psi$$

Thus,

$$\frac{512 \cdot 10^{-3} \cdot (0.1)^2 \cdot (10^{-3})^2}{34 \cdot 10^6 \cdot (0.5 \cdot 10^{-3})^2 \, [(1.10^{-3})^2 - (0.5 \cdot 10^{-3})^2]} = 8.10^{-4} < 0.01$$

Accordingly, a catheter 10 having the above dimensions would satisfy the requirement for low system compliance, allowing the use of such a construct for the measurement of intra-ventricular pressure without loss of signal fidelity.

A person skilled in the art will appreciate that a variety of factors should be considered in optimizing the system to provide an accurate pressure reading.

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

**Claims**

1. A pressure sensor device, comprising:

an elongate catheter having

a first lumen adapted to accommodate fluid flow therethrough; and

a second, separate, fluid-filled, fluid-impermeable, sealed lumen extending between a pressure-sensitive component adapted to be exposed to an external pressure source, and a pressure sensor that is effective to measure pressure of the external pressure source in response to displacement of the pressure-sensitive component.

2. The device of claim 1, wherein the elongate catheter includes a sidewall extending between proximal and distal ends, and the first lumen extends through the elongate catheter and includes at least one fluid-entry port formed through the sidewall at or adjacent to a distal end of the catheter.

3. The device of claim 1, wherein the pressure-sensitive component is disposed at a distal end of the second lumen, and the pressure sensor is coupled to a proximal end of the second lumen.

4. The device of claim 1, wherein the pressure-sensitive component includes a first surface in contact with fluid within the second lumen, and a second, opposed surface adapted to be exposed to an external pressure source.

5. The device of claim 4, wherein the pressure-sensitive component comprises a flexible membrane.

6. The device of claim 5, wherein the flexible membrane is disposed across an opening formed in the sidewall of the catheter.

7. The device of claim 5, wherein the flexible membrane has a compliance that is in the range of about 0.05 μL/mmHg to 2 μL/mmHg.

8. The device of claim 5, wherein the flexible membrane is formed from a material selected from the group consisting of polyurethane, silicone, and solvent-based polymer solutions.

9. The device of claim 1, wherein the second lumen contains a predetermined volume of fluid.

10. The device of claim 9, wherein the second lumen is free of voids.

11. The device of claim 9, wherein the volume of fluid in the second lumen is in the range of about 1 μL to 10 μL.

12. The device of claim 1, wherein the fluid in the second lumen is a low viscosity silicone fluid.

13. The device of claim 1, wherein the fluid in the second lumen is a biocompatible fluid.

14. The device of claim 1, wherein the fluid in the second lumen has an average kinematic viscosity in the range of about 5 cs to 20 cs.

15. The device of claim 1, wherein the second lumen has a diameter that is less than a diameter of the first lumen.

16. The device of claim 1, wherein the second lumen has a diameter that is in the range of about 0.1 mm to 0.3 mm, and the second lumen has a length that is in the range of about 8 cm to 20 cm.

17. The device of claim 1, wherein the catheter has a compliance that is less than a compliance of the pressure-sensitive component.

18. The device of claim 1, wherein the catheter has a low compliance such that it is not susceptible to deformation as a result of exposure to the external pressure source.

19. The device of claim 1, wherein the pressure sensor has a frequency response that is greater than 20 Hz.

20. The device of claim 1, wherein the pressure sensor has a compliance that is in the range of about 0.1 μL/mmHg to 0.02 μL/mmHg.

21. The device of claim 1, wherein the pressure-sensitive component comprises a flexible sleeve that is formed around a distal end of the catheter and that is in fluid communication with the second lumen.

**22.** An intra-ventricular catheter, comprising:

an elongate member having a first lumen adapted to accommodate fluid flow therethrough, and a second, fluid-sealed lumen having a pressure sensor coupled to a flexible membrane disposed at a distal end of the catheter and that is adapted to respond to intra-ventricular pressure changes when the catheter is implanted within a patient's ventricle such that direct pressure readings of the intra-ventricular pressure can be measured.

**23.** The intra-ventricular catheter of claim 22, wherein the pressure sensor is coupled to a proximal end of the second, fluid-sealed lumen.

**24.** The intra-ventricular catheter of claim 23, wherein the flexible membrane is formed across a discontinuity formed in a sidewall of the catheter.

**25.** The intra-ventricular catheter of claim 22, wherein the flexible membrane has a compliance that is in the range of about 0.05 µL/mmHg to 2 µL/mmHg.

**26.** The intra-ventricular catheter of claim 22, wherein the second lumen contains fluid having a low viscosity.

**27.** The intra-ventricular catheter of claim 22, wherein the pressure sensor has a frequency response that is greater than 20 Hz.

**28.** The intra-ventricular catheter of claim 22, wherein the pressure-sensitive component comprises a flexible sleeve that is formed around a distal end of the catheter and that is in fluid communication with the second lumen.

**29.** A method for measuring intra-ventricular pressure, comprising:

providing a ventricular catheter having

a first lumen adapted to accommodate fluid flow therethrough, and
a second, fluid-sealed, fluid-impermeable lumen extending between a distal, pressure-sensitive member adapted to respond to pressure changes in a patient's ventricle, and a proximal pressure sensor adapted to measure the pressure changes;

implanting the ventricular catheter in a patient's ventricle such that the pressure-sensitive member is disposed within the ventricle and the pressure sensor is disposed at a location outside of the ventricle; and
obtaining at least one reading of the pressure within the patient's ventricle.

**30.** The method of claim 29, wherein the pressure-sensitive member comprises a flexible membrane that is formed across a discontinuity formed in a sidewall of the catheter.

**31.** The method of claim 30, wherein the flexible membrane has a compliance that is in the range of about 0.05 µL/mmHg to 2 µL/mmHg.

**32.** The method of claim 29, wherein the second lumen contains fluid having a low viscosity.

**33.** The method of claim 29, wherein the pressure sensor has a frequency response that is greater than about 20 Hz.

**34.** A method of manufacturing an intra-ventricular pressure sensor device, comprising:

forming a catheter having a first lumen adapted to receive fluid flow therethrough, and a second lumen extending between a proximal, pressure sensor and a distal end in communication with an opening formed in a sidewall of the catheter;
filling the second lumen of the catheter with fluid;
spraying a solvent-based silicone solution over the opening formed in the sidewall of the catheter to form a flexible membrane that is effective to seal the fluid within the second lumen in the catheter.

**35.** The method of claim 34, further comprising the step of removing any voids in the second lumen after the second lumen is filled with fluid.

**FIG. 1**

10

14

10a

TO
SENSOR
(22)

14a

16

12a

TO VALVE
OR OTHER
COMPONENT
(50)

14b

12

18

20

18

12b

10b

**FIG. 2**

10

$l_2$

10b

14b

16

24

14

$d_2$

14a

22

12b

$d_1$

20

18

18

18

12

12a

10a

$l_1$

**FIG. 3**

$10'$

$12'$

$24'$

$24'$

$14'$

$16'$

$18'$

$20'$

## PARTIAL EUROPEAN SEARCH REPORT

**European Patent Office**

which under Rule 45 of the European Patent Convention shall be considered, for the purposes of subsequent proceedings, as the European search report

**Application Number**

EP 04 25 5053

### DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | US 5 573 007 A (BOBO SR DONALD E) 12 November 1996 (1996-11-12) * column 16, line 15 - column 17, line 20; figures 6,6B * | 1-6,9, 22-24,28 | A61B5/03 |
| X | DE 100 33 138 A (GLUKOMEDITECH AG) 31 January 2002 (2002-01-31) * column 5, lines 19-35; figure 2 * | 22 | |
| A | US 5 964 714 A (LAFONTAINE DANIEL M) 12 October 1999 (1999-10-12) * the whole document * | 1-33 | |
| A | US 6 156 064 A (CHOUINARD PAUL F) 5 December 2000 (2000-12-05) * column 9, line 57 - column 10, line 24 * | 34 | |

**TECHNICAL FIELDS SEARCHED (Int.Cl.7)**

A61B
B01D

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

| Place of search | Date of completion of the search | Examiner |
|---|---|---|
| Munich | 11 January 2005 | Schoeffmann, H |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

| European Patent Office | INCOMPLETE SEARCH SHEET C | Application Number EP 04 25 5053 |

Claim(s) searched completely:
   1-28,34,35

Claim(s) not searched:
   29-33

Reason for the limitation of the search (non-patentable invention(s)):

Method of treatment having surgical character (Art.52(4) EPC)

**ANNEX TO THE EUROPEAN SEARCH REPORT
ON EUROPEAN PATENT APPLICATION NO.**                    EP 04 25 5053

This annex lists the patent family members relating to the patent documents cited in the above-mentioned European search report.
The members are as contained in the European Patent Office EDP file on
The European Patent Office is in no way liable for these particulars which are merely given for the purpose of information.

11-01-2005

| Patent document cited in search report | | Publication date | Patent family member(s) | | Publication date |
|---|---|---|---|---|---|
| US 5573007 | A | 12-11-1996 | DE | 69528767 D1 | 12-12-2002 |
| | | | DE | 69528767 T2 | 19-05-2004 |
| | | | EP | 0774919 A1 | 28-05-1997 |
| | | | WO | 9604846 A1 | 22-02-1996 |
| DE 10033138 | A | 31-01-2002 | DE | 10033138 A1 | 31-01-2002 |
| | | | AU | 7249201 A | 21-01-2002 |
| | | | WO | 0203860 A1 | 17-01-2002 |
| US 5964714 | A | 12-10-1999 | AT | 253322 T | 15-11-2003 |
| | | | DE | 69725949 D1 | 11-12-2003 |
| | | | DE | 69725949 T2 | 02-09-2004 |
| | | | EP | 0836410 A1 | 22-04-1998 |
| | | | WO | 9732518 A1 | 12-09-1997 |
| | | | US | 5860938 A | 19-01-1999 |
| US 6156064 | A | 05-12-2000 | AT | 280547 T | 15-11-2004 |
| | | | CA | 2340439 A1 | 24-02-2000 |
| | | | DE | 69921481 D1 | 02-12-2004 |
| | | | EP | 1472994 A2 | 03-11-2004 |
| | | | EP | 1104266 A1 | 06-06-2001 |
| | | | JP | 2002522155 T | 23-07-2002 |
| | | | WO | 0009041 A1 | 24-02-2000 |
| | | | US | 6709455 B1 | 23-03-2004 |
| | | | US | 2004167606 A1 | 26-08-2004 |

EPO FORM P0459

For more details about this annex : see Official Journal of the European Patent Office, No. 12/82